# EUROPEAN PATENT APPLICATION

(11) **EP 4 563 196 A1**
(43) Date of publication of application: **04.06.2025**
(21) Application number: 23945223.8
(22) Date of filing: 13.10.2023
(51) Int. Cl.: A61N 5/10

(54) **LOW-DOSE RADIATION TREATMENT SYSTEM FOR TREATING DEMENTIA AND CHRONIC INFLAMMATION USING DIFFERENT PHOTON ENERGY LEVELS**

(30) Priority: 13.07.2023 KR 20230091275
(71) Applicant: ReadyCure Inc., Seoul 02792 (KR)
(72) Inventor: CHUNG, Weon Kuu, Seoul 06288 (KR)
(74) Representative: Prüfer & Partner mbB Patentanwälte · Rechtsanwälte
(86) International application number: PCT/KR2023/015874
(87) International publication number: WO 2025/013994

(57) **Abstract**

Provided is a low-dose radiation treatment system for dementia or chronic inflammation treatment based on a photon energy level, including: a low-dose radiation therapy device wherein a treatment bed where a patient receives treatment while lying down is formed on a device body and a plurality of carbon nanotube sources are installed around the inner periphery of a ring-shaped gate of the device body to form a radiation gantry so that therapeutic radiation is irradiated toward the patient's body; and a controller configured to check a patient's condition using entered patient information, to set a treatment direction and to perform radiation therapy by driving the treatment bed such that a patient is positioned at an optimal treatment position and by controlling the operation of the low-dose radiation therapy device, wherein the plural carbon nanotube sources are installed around the inner periphery of the radiation gantry, and the carbon nanotube sources are distributed by band in a 100 to 300 kVp range, and the controller may selectively use radiation in a specific area depending upon a patient's disease type.

## Description

### [Cross-Reference to Related Application]

This application is a National Stage Entry of PCT International Application No. PCT/KR2023/015874, which was filed on October 13, 2023, and which claims priority to Korean Patent Application No. 10-2023-0091275, filed on July 13, 2023 in the Korean Intellectual Property Office, the disclosure of which is incorporated herein by reference.

### [Technical Field]

The present invention relates to a low-dose radiation treatment system for dementia or chronic inflammation treatment based on a photon energy level, and more particularly to a low-dose radiation treatment system for dementia or chronic inflammation treatment based on a photon energy level which is advantageous for the treatment of chronic inflammation such as dementia sensitive to radiation exposure wherein a plurality of carbon nanotube sources are installed around the inner periphery of a radiation gantry, the carbon nanotube sources are distributed by band in a 100 to 300 kVp range, and a controller selectively uses radiation in a specific area depending upon a patient's disease type.

### [Background Art]

Among the types of radiation generally used for medical purposes, therapeutic radiation is applied to the tumor of a cancer patient and is used to prevent cancer cells from reproducing further, causing the cancer cells to die at the end of their lifespan or relieving the patient's pain.

Recently, Intensity Modulated Radiation Therapy (IMRT) of modulating the intensity of radiation to suit the shape, size, and location of tumor using the MultiLeaf Collimator (MLC) to reduce side effects caused by radiation in normal tissue and maximize treatment results by irradiating radiation with optimal energy has attracted attention.

However, there is a problem in that treatment devices used in such radiation therapy are limitedly used only for cancer treatment.

### [Disclosure]

### [Technical Problem]

Therefore, the present invention has been made in view of the above problems, and it is one object of the present invention to provide a low-dose radiation treatment system for dementia or chronic inflammation treatment based on a photon energy level, the low-dose radiation treatment system including a low-dose radiation therapy device configured to form a radiation gantry so that therapeutic radiation is irradiated toward the patient's body by installing a plurality of carbon nanotube sources around the inner periphery of a ring-shaped gate, wherein an optimized radiation treatment environment tailored to a patient is provided by checking a patient's condition and setting a treatment direction using patient information entered through a controller, and then controlling the operation of the low-dose radiation therapy device to perform radiation therapy and adjust the size of radiation energy irradiated from the radiation therapy device according to the patient's individual characteristics and a treatment purpose, an angle at which the radiation is irradiated, and the radiation dose.

It is another object of the present invention to provide a low-dose radiation treatment system for dementia or chronic inflammation treatment based on a photon energy level which is advantageous for the treatment of chronic inflammation such as dementia sensitive to radiation exposure wherein a plurality of carbon nanotube sources are installed around the inner periphery of a radiation gantry, the carbon nanotube sources are distributed by band in a 100 to 300 kVp range, and a controller selectively uses radiation in a specific area depending upon a patient's disease type.

It is yet another object of the present invention to provide a low-dose radiation treatment system for dementia or chronic inflammation treatment based on a photon energy level which can perform patient-tailored treatment by establishing a precise radiation treatment plan, is safe because it reduces the risk of radiation exposure, and can be used to treat benign keloids, arthritis, heterotopic bone ossification, psoriasis, atopy, dementia, Parkinson's disease, and animal cancer as well as skin cancer.

### [Technical Solution]

In accordance with an aspect of the present invention, the above and other objects can be accomplished by the provision of a low-dose radiation treatment system for dementia or chronic inflammation treatment based on a photon energy level, including: a low-dose radiation therapy device wherein a treatment bed where a patient receives treatment while lying down is formed on a device body and a plurality of carbon nanotube sources are installed around the inner periphery of a ring-shaped gate of the device body to form a radiation gantry so that therapeutic radiation is irradiated toward the patient's body; and a controller configured to check a patient's condition using entered patient information, to set a treatment direction and to perform radiation therapy by driving the treatment bed such that a patient is positioned at an optimal treatment position and by controlling the operation of the low-dose radiation therapy device, wherein the plural carbon nanotube sources are installed around the inner periphery of the radiation gantry, and the carbon nanotube sources are distributed by band in a 100 to 300 kVp range, and the controller may selectively use radiation in a specific area depending upon a patient's disease type.

Here, the controller may control a preset dose, energy intensity and a beam irradiation range and angle depending upon a patient's disease type.

In addition, a plurality of carbon nanotube sources may be arranged in the inner periphery of the radiation gantry, the carbon nanotube sources may be distributed along the arc radius, and each of the carbon nanotube sources may emit a radiation beam toward a concentric center of an arc.

Here, the radiation gantry may emit a dose of 10 to 100 cGy/min to the installed carbon nanotube sources, and the controller may enable a preset dose to be irradiated depending upon a patient's disease type.

In addition, the plural carbon nanotube sources may emit radiation beams sequentially or alternately.

In addition, radiation beam energy irradiated from the radiation gantry may be set to a pulsed beam energy of 100~300 kVp and 3mA, and a radiation time (beam on-off time) of the radiation beam may be set to 1 minute or less.

In addition, the low-dose radiation therapy device may include: a treatment bed where a patient lies down and receives treatment; a device body where the treatment bed is installed to reciprocate in a longitudinal direction; and a radiation gantry installed in an inner periphery direction of the gate such that therapeutic radiation is irradiated toward patient's body, wherein a ring-shaped gate is formed on an upper side of the device body so that the bed passes through a center thereof.

Here, a rotator for rotating the radiation gantry around the patient's body to change a radiation irradiation position may be further included.

In addition, carbon nanotube sources may be arranged in multiple rows around the inner periphery of the gate of the radiation gantry, wherein the carbon nanotube source arrays operate alternatively.

### [Advantageous effects]

The present invention provides a low-dose radiation treatment system for dementia or chronic inflammation treatment based on a photon energy level, the low-dose radiation treatment system including a low-dose radiation therapy device configured to form a radiation gantry so that therapeutic radiation is irradiated toward the patient's body by installing a plurality of carbon nanotube sources around the inner periphery of a ring-shaped gate, wherein an optimized radiation treatment environment tailored to a patient is provided by checking a patient's condition and setting a treatment direction using patient information entered through a controller, and then controlling the operation of the low-dose radiation therapy device to perform radiation therapy and adjust the size of radiation energy irradiated from the radiation therapy device according to the patient's individual characteristics and a treatment purpose, an angle at which the radiation is irradiated, and the radiation dose.

In addition, the present invention is advantageous for the treatment of chronic inflammation such as dementia sensitive to radiation exposure because a plurality of carbon nanotube sources are installed around the inner periphery of a radiation gantry, the carbon nanotube sources are distributed by band in a 100 to 300 kVp range, and a controller selectively uses radiation in a specific area depending upon a patient's disease type.

Further, the present invention can perform patient-tailored treatment by establishing a precise radiation treatment plan, is safe because it reduces the risk of radiation exposure, and can be used to treat benign keloids, arthritis, heterotopic bone ossification, psoriasis, atopy, dementia, Parkinson's disease, and animal cancer as well as skin cancer or to treat acute or chronic pain in the musculoskeletal, spine, etc. and chronic inflammation that is difficult to control with medication.

### [Description of Drawings]

FIG. 1 is a block diagram for explaining a low-dose radiation treatment system according to the present invention.
FIG. 2 is a conceptual diagram for explaining the arrangement structure of carbon nanotube sources of the low-dose radiation therapy device according to the present invention.
FIG. 3 illustrates the progress of concurrent drug treatment and radiation treatment on experimental mice according to an existing method.
FIG. 4 illustrates the progress of concurrent treatment for experimental mice according to a low-dose radiation treatment system of the present invention.
FIG. 5 illustrates Aβ cortex images and the number of plaques per unit area (mm³), compared to a control, according to the radiation therapy result of the existing method.
FIG. 6 illustrates Aβ cortex images and the number of plaques per unit area (mm³), compared to a control, according to the low-dose radiation therapy result of the present invention.
FIG. 7 illustrates a relative expression of the radiation therapy result of the existing method to a control group through RT-PCR.
FIG. 8 illustrates a relative expression of the low-dose radiation therapy result of the present invention to a control group through RT-PCR.
FIG. 9 illustrates a perspective view of the low-dose radiation therapy device according to the present invention.
FIG. 10 illustrates a front view of the low-dose radiation therapy device according to the present invention.
FIG. 11 illustrates a side view of the low-dose radiation therapy device according to the present invention.

### [Best Mode]

Hereinafter, the present invention according to a preferred embodiment will be described in detail with reference to the attached drawings. Here, the same symbols are used for the same components, and repetitive descriptions and detailed descriptions of known functions and configurations that may unnecessarily obscure the gist of the invention are omitted. Embodiments of the invention are provided to more completely explain the present invention to those with average knowledge in the art. Therefore, the shapes and sizes of elements in the drawings may be exaggerated for clearer explanation.

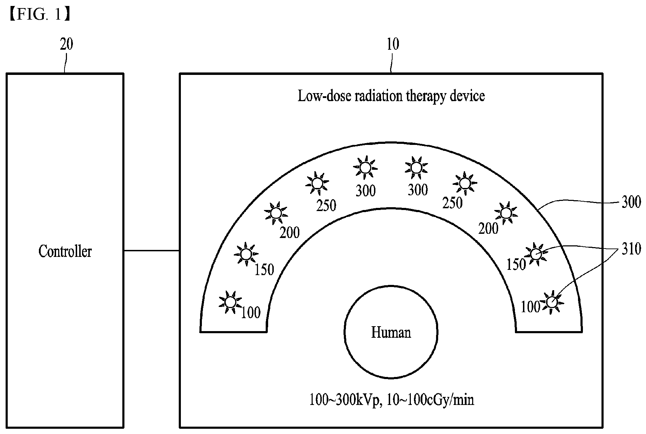

FIG. 1 is a block diagram for explaining a low-dose radiation treatment system according to the present invention.

Referring to FIG. 1, a low-dose radiation treatment system according to the present invention includes a low-dose radiation therapy device 10 wherein a treatment bed 100 where a patient receives treatment while lying down is formed on a device body 200 and a plurality of carbon nanotube sources 310 are installed around the inner periphery of a ring-shaped gate of the device body 200 to form a radiation gantry 300 so that therapeutic radiation is irradiated toward the patient's body; and a controller 20 configured to check a patient's condition using entered patient information, to set a treatment direction and to perform radiation therapy by driving the treatment bed such that a patient is positioned at an optimal treatment position and by controlling the operation of the low-dose radiation therapy device 10.

Here, the plural carbon nanotube sources 310 are installed around the inner periphery of the radiation gantry 300, and the carbon nanotube sources 310 are distributed by band in a 100 to 300 kVp range, and the controller 20 may selectively use radiation in a specific area depending upon a patient's disease type.

The controller 20 may control a preset dose, energy intensity, and a beam irradiation range and angle depending upon a patient's disease type.

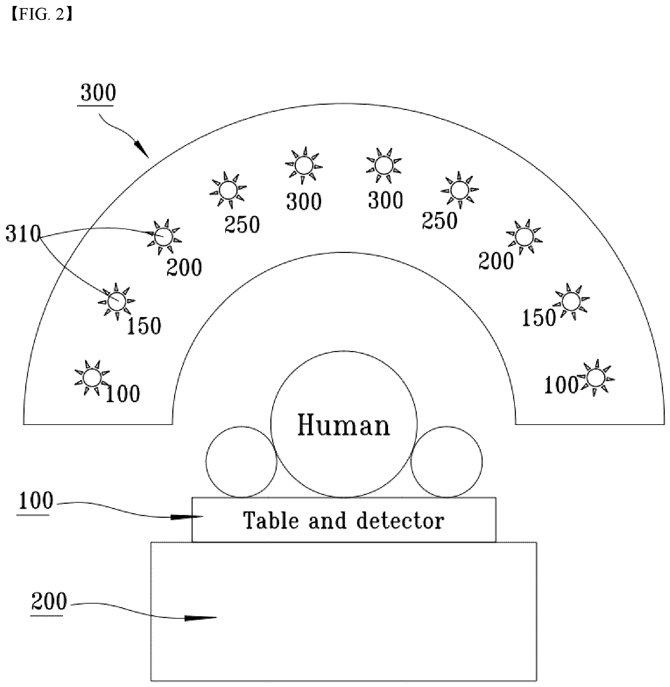

FIG. 2 is a conceptual diagram for explaining the arrangement structure of carbon nanotube sources of the low-dose radiation therapy device according to the present invention. Referring to FIG. 2, the plural carbon nanotube sources 310 are formed on the inner periphery of the radiation gantry 300 of the present invention, the carbon nanotube sources 310 are distributed along the arc radius, and each of the carbon nanotube sources 310 emits a low-dose radiation beam toward the concentric center of the arc.

As shown in FIG. 2, the radiation gantry 300 may emit a dose of 10 to 100 cGy/min to the installed carbon nanotube sources, and the controller 20 may enable a preset dose to be irradiated depending upon a patient's disease type.

Here, the radiation beam energy irradiated from the radiation gantry 300 may be set to a pulsed beam energy of 100 to 300 kVp and 3 mA, and the radiation beam radiation time (beam on off time) may be set to 1 minute or less.

Here, the radiation beam radiation time (beam on off time) may be controlled to a unit of 1 msec (1/1000th of a second) or less. In addition, a radiation beam may be irradiated by turning the carbon nanotube sources 310 on/off in a 1 msec (1/1000th of a second) unit during the radiation beam radiation time.

The present invention may control the radiation beam irradiation interval between the carbon nanotube sources 310 to the cell damage repair time (DNA repair time), which is capable of repairing DNA damage in normal cells, while minimizing the radiation beam radiation time (beam on-off time).

As described above, the present invention allows the plural carbon nanotube sources 310 to sequentially or alternately irradiate radiation beams. Accordingly, the lifespan of the carbon nanotube sources 310 can be extended by preventing deterioration thereof, and can shorten a time required for overall treatment by shortening a waiting time until the next radiation, enabling intensive radiation therapy.

Here, low-dose radiation (LDR) refers to low-intensity radiation that is close to natural. A large amount of radiation may harm living organisms, but this small amount of low-dose radiation actually promotes the physiological activities of living organisms, extending lifespan, promoting growth, or lowering the tumor incidence rate. This is called radiation hormesis.

It has been reported that low-dose radiation in the miligray (mGy) range induces resistance to disease and exhibits an adaptive protection effect, unlike high-dose radiation (Sakai K., et al., Dose Response, 2006, 4(4):327~332; Sakai K., et al, Int. J. Low Radiat., 2003, 1(1):142~146; Scott BR., Dose Response, 2008 6(3):299~318), and there is a report that when a certain amount of low-dose radiation was irradiated to experimental mice with diabetes genes, their condition was improved (Sakai K., et al., Dose Response, 2006, 4(4):327~332). In addition, it was reported that radiation below 0.25Gy is effective in protecting against DNA damage and repairing damaged DNA (Le XC., et al., Science, 1998, 280(5366):1066~1069) and low-dose radiation has an immune-boosting effect (Nogami M., et al., Int. J. Radiat. Biol., 1993, 63(6):775~783; Nogami M., et al., Radiat. Res., 1994, 139(1):47~52).

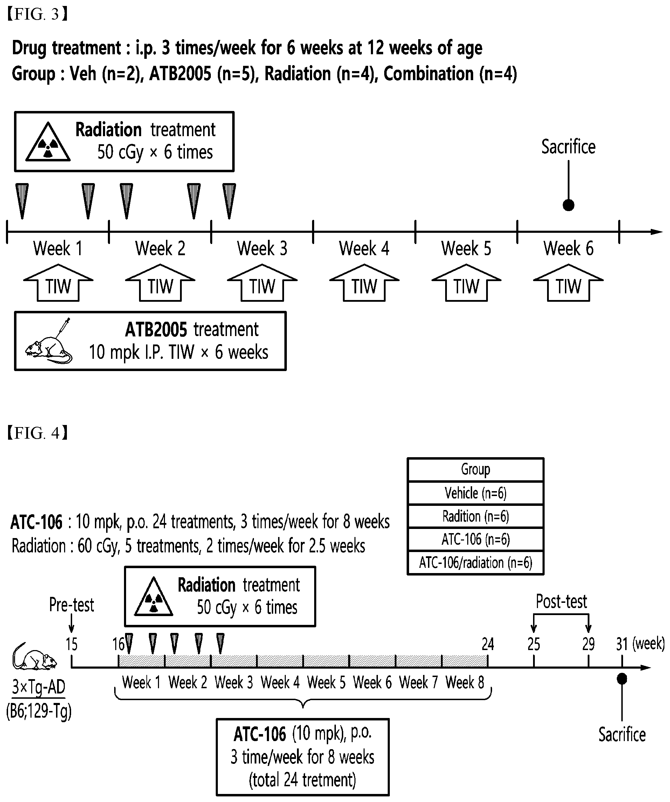

FIG. 3 illustrates the progress of concurrent drug treatment and radiation treatment on experimental mice according to an existing method.

Referring to FIG. 3, when a combination of drug treatment, radiation therapy, and ATB2005 treatment was administered to 12-week-old experimental mice according to the existing method, the mice survived until the 6th week. Specifically, drug treatment was administered 3 times a week for 6 weeks, radiation therapy was administered at 50 cGy x 5 times, ATB2005 treatment was performed with 10mpk I.P., and TIW was conducted for 6 weeks.

FIG. 4 illustrates the progress of concurrent treatment for experimental mice according to a low-dose radiation treatment system of the present invention.

Referring to FIG. 4, the low-dose radiation treatment system of the present invention was used, 12-week-old experimental mice were used as subjects, ATC-108 (10 mpk) treatment was performed a total of 24 times at intervals of 3 times a week for 8 weeks, and radiation therapy was administered 5 times in total with low-dose radiation of 100 to 300 kVp and 60 cGy at intervals of 2 times a week for 2.5 weeks. As a result, the experimental mice survived until the 31st week.

When comparing the experimental result (6-week survival) according to the existing method of FIG. 3 with the experimental result (31-week survival) according to the low-dose radiation treatment system of the present invention of FIG. 4, it can be seen that the survival rate of mice was significantly superior in the low-dose radiation treatment system of the present invention.

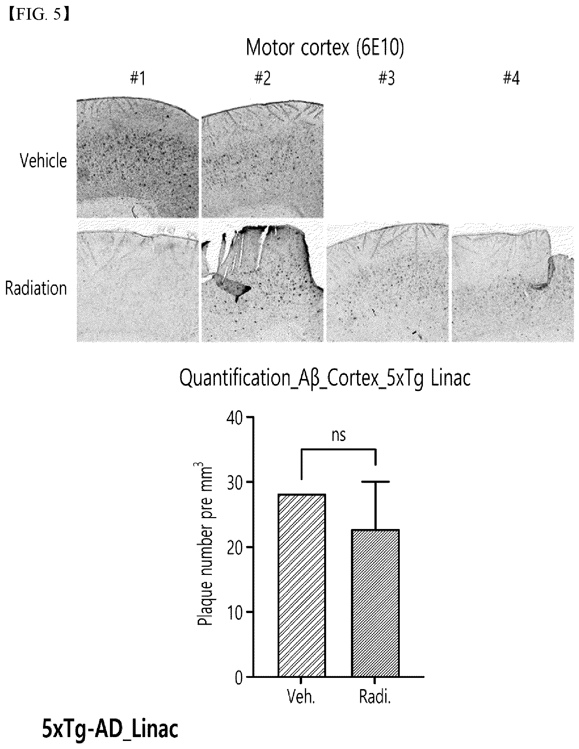

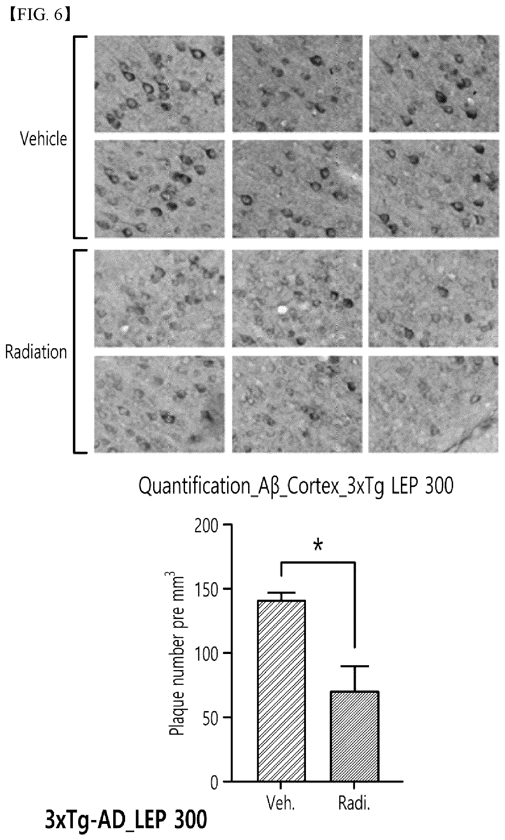

FIG. 5 illustrates Aβ cortex images and the number of plaques per unit area (mm³), compared to a control, according to the radiation therapy result of the existing method, and FIG. 6 illustrates Aβ cortex images and the number of plaques per unit area (mm³), compared to a control, according to the low-dose radiation therapy result of the present invention.

Comparing FIGS. 5 and 6, FIG. 5 shows a treatment result using existing 6MeV radiation. From this, it can be seen that the number of plaques per unit area (mm³) to the control group (Vehicle) not receiving radiation therapy was 28:22 which was not a significantly reduced ratio.

On the other hand, from the treatment result of FIG. 6 using the low-dose radiation (i.e., 300 kVp radiation) therapy result of the present invention, it can be seen that the number of plaques per unit area (mm³) to the control group (Vehicle) not receiving radiation therapy was 140 : 70 which was a significantly reduced ratio.

As shown in the results of FIGS. 5 and 6 compared with the control, the decrease in the number of plaques per area (mm³) when with a low-dose radiation of kVp unit according to the present invention was greater than when irradiated with a dose of MeV unit according to the existing method. Here, the number of plaques was counted by staining amyloid beta (Aβ) cells and was proportional to the degree of dementia.

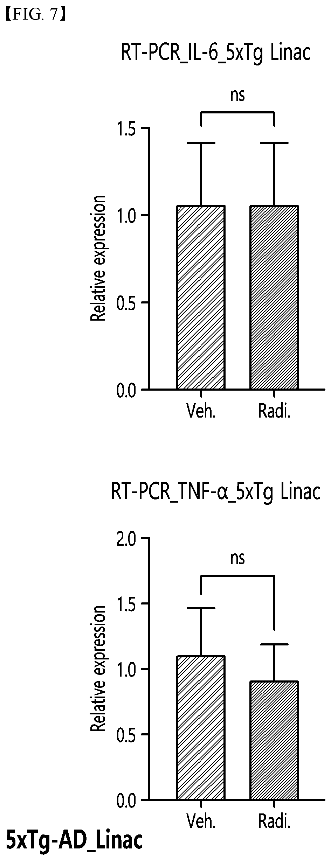

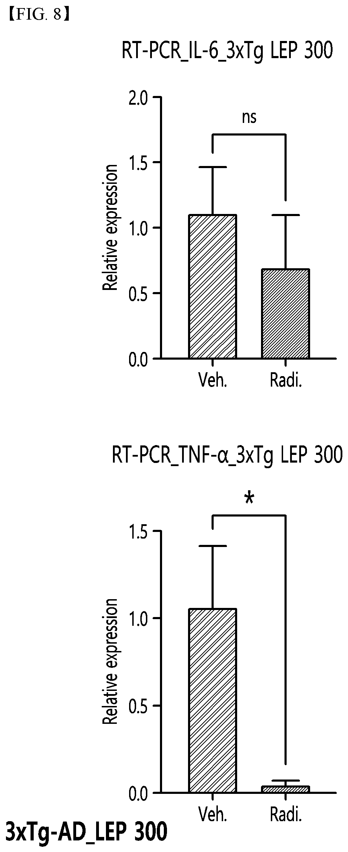

FIG. 7 illustrates a relative expression of the radiation therapy result of the existing method to a control group through RT-PCR, and FIG. 8 illustrates a relative expression of the low-dose radiation therapy result of the present invention to a control group through RT-PCR.

As shown in FIG. 7, the existing radiation therapy result was subjected to RT-PCR_TNF-a and RT-PCR_IL-6 experiments. As a result, it can be seen that the relative expression ratios compared to the control group were 1: 1 and 1:0.8, respectively, showing almost no difference in the ratio.

As shown in FIG. 8, the low-dose radiation therapy result of the present invention was also subjected to RT-PCR_TNF-a and RT-PCR_IL-6 experiments. As a result, it can be seen that the relative expression ratios compared to the control group were 1.2:0.6 and 1:0.1, respectively, showing a ratio difference of more than 2 times.

Recently, several preclinical and exploratory clinical research papers have reported that low-dose radiation has anti-inflammatory and neuroprotective effects, which are explained by the mechanism of inhibiting the NFkb pathway and regulating microglial cell phenotype.

In a recent research result, it was reported that dementia patients with clinically more severe disease were irradiated three times with 120 kVp X-ray energy (brain computed tomography CT), and three out of four patients who received 16cGy, i.e. 75%, showed remarkable improvement in cognitive function (source: low dose of ionizing radiation as a treatment for Alzheimer's disease : journal of Alzheimer's disease 2021).

In addition, it was reported that, in patients with osteoarthritis, the pain reduction treatment effect was 13-21% superior when 120 to 300 kVp, where the photoelectric effect is dominant, was used, compared to when radiation was used in the area where the Compton effect (6MeV) is dominant (source: Strahlentherapie und Onkologie volume 196, pages715-724 (2020).

In previous studies, it was reported that the quality of the radiation (electron, proton, alpha particle) did not have a significant effect on telomere shortening when the same dose of radiation is irradiated, but, in chromosome aberration and apoptosis, the biological effects of high LET protons and baryons were significantly greater than those of low LET radiation.

Therefore, when radiation in the region of 120-300 kVp, where the photoelectric effect is physically dominant, and radiation in the region, where Compton effect (6 MeV) is dominant, are compared in terms of radiobiological effects, it can be seen that the RadioBiologic Effect (RBE) is twice as large as 6 MeV (2 to1), and it can be predicted that energy of 120-300 kVp, where the energy dissipates quickly, is more effective in terms of biological effects, especially cell protection and anti-inflammatory effects, in low-dose areas.

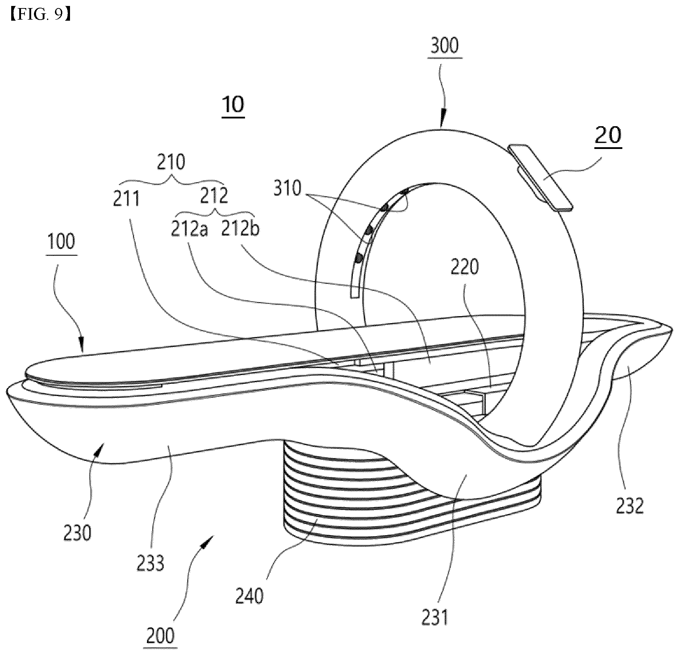

Hereinafter, a preferred embodiment of the low-dose radiation therapy device according to the present invention is described in detail with reference to FIGS. 9 to 11.

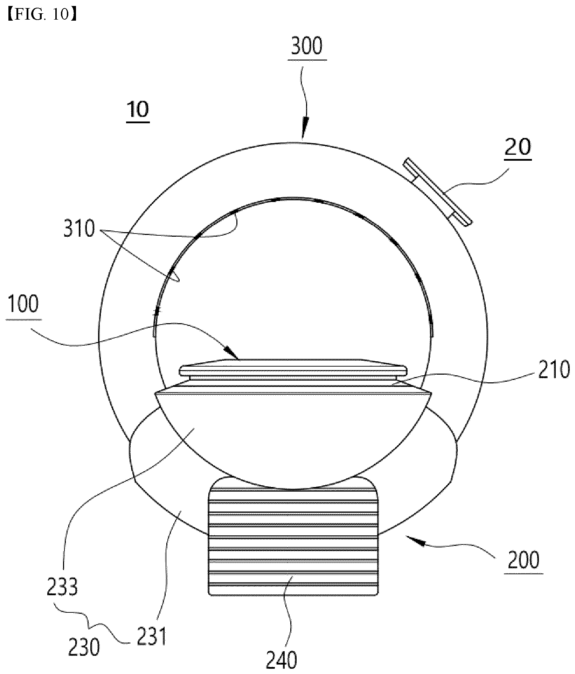

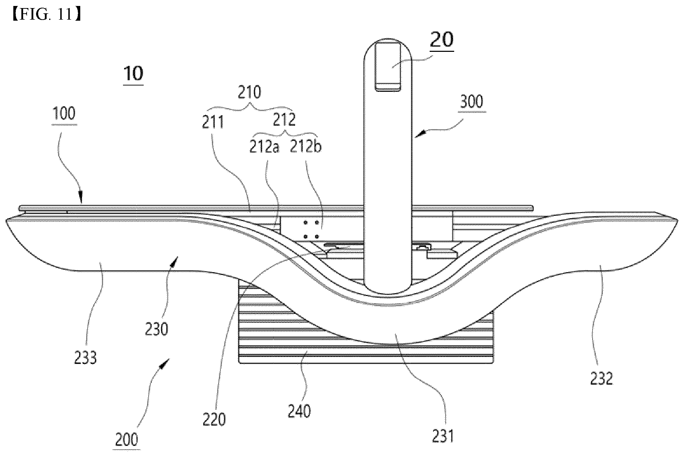

FIG. 9 illustrates a perspective view of the low-dose radiation therapy device according to the present invention, FIG. 10 illustrates a front view of the low-dose radiation therapy device according to the present invention, and FIG. 11 illustrates a side view of the low-dose radiation therapy device according to the present invention.

Referring to FIGS. 9 to 11, a low-dose radiation therapy device 10 according to the present invention includes a bed 100, a device body 200, and a radiation gantry 300.

First, the bed 100 may be provided a plank-shaped structure extending longitudinally so that a patient can receive treatment while lying down. Here, opposite ends of the bed 100 may be formed in a round shape, contributing to a patient-friendly design.

The bed 100 may be moved back and forth through a sliding operation in the longitudinal direction while a patient is lying down, so that the treatment area of a patient can be positioned at the center of the ring-shaped radiation gantry 300.

A sliding driver 210 is provided for the sliding operation of the bed 100. The sliding driver 210 is installed on the device body 200 to support the bed 100.

Hereinafter, the device body 200 is described in detail. The device body 200 includes a sliding driver 210, a radiation detector 220 and the main body case 230.

The sliding driver 210 is installed on the transfer path of the bed 100, and supports a lower part of the bed 100 such that the bed 100 can slidably move. The sliding driver 210 includes a support 211 formed under the bed 100 to face the bed 100.

Here, the support 211 has a plate shape extending in a longitudinal direction. Opposite ends of the support 211 are coupled to and supported by the top of the main body case 230.

Here, a rail 212 for slidingly guiding the bed 100 is formed on the support 211.

The rail 212 may include a pair of side fixing rails 212a respectively installed on the left and right sides in the longitudinal direction of the support 211; and a rail guide 212b rail-coupled to and slid on the side fixing rails 212a.

Here, the bed 100 is connected to the top of the rail guide 212b so that the bed 100 is slidably driven together with the rail guide 212b.

Meanwhile, the support 211 may include a driver (not shown) for reciprocating the bed 100 in the rail direction. Here, the driver may use an actuator such as a motor or hydraulic cylinder as a power source.

In addition, the radiation detector 220 is formed under the sliding driver 210. The radiation detector 220 is a detection device that detects radiation that has passed through a subject.

A signal detected through the radiation detector 220 may be provided on an image or screen through a display device. Here, the radiation detector 220 may be installed in the main body case 230.

Hereinafter, the main body case 230 is described in detail. The sliding driver 210 and the radiation detector 220 are placed in the main body case 230, and the main body case 230 forms the exterior of the device body.

In addition, the main body case 230 includes a center part 231 where the radiation gantry 300 and the radiation detector 220 are combined, a headrest 232 whose one end floats in the air extends from the center 231, and a leg support 233 is formed at an end on the opposite side of the headrest 232 to float in the air.

Here, the opposite ends of the support 211 of the sliding driver are respectively coupled to the headrest 232 and leg support 233 of the main body case 230.

Here, the opposite ends of the main body case 230 connected to the support 211 may be formed in a circular round shape. This circular round design creates a friendly and soft feeling, contributing to the psychological stability of a patient.

The center part 231 of the main body case 230 has a shape curved in a bow shape toward the bottom.

Meanwhile, as the center part 231 of the main body case 230 is curved downward, a certain section on the side of the support 211 and a space of the radiation detector 220 below may be partially opened.

Here, the radiation gantry 300 is coupled to opposite ends of the radiation detector 220.

The concept of exposing the internal structure of the device has the advantage of highlighting the simplicity and stability of the device. In addition, the interior is exposed, making maintenance of the structure convenient.

Meanwhile, the bottom of the center part 231 may be fixed to the floor of a building using the holder 240.

Inside the holder 240, electrical equipment, control equipment, and communication equipment for driving an ionizing radiation therapy device may be installed.

On the device body 200, the radiation gantry 300 for radiating therapeutic radiation to the patient's torso is installed.

Hereinafter, the radiation gantry 300 is described in detail. The radiation gantry 300 forms a ring-shaped gate that surrounds the patient's torso. Here, a plurality of carbon nanotube sources 310 for radiating therapeutic radiation toward the patient's torso are formed in the inner periphery of the gate.

More particularly, the plural carbon nanotube sources 310 configured to emit low energy of 100 to 300 kVp (kilovolt peak) and low-dose radiation of 10 to 100 cGy/min may be distributed by band around the inner periphery of the radiation gantry 300.

**In** addition, the treatment bed 100 according to the present invention may further include a rotator (not shown) for rotating the patient's body along the inner periphery of the radiation gantry 300.

For example, the rotator (not shown) rotates a patient and the treatment bed 100 while the patient is lying down to change the position of the patient's body part to which radiation is irradiated, allowing more active control of the radiation irradiation range and intensity depending upon the patient's body part.

In addition, the carbon nanotube sources 310 are arranged in multiple rows around the inner periphery of the gate of the radiation gantry 300, and each array of the carbon nanotube sources 310 may be operated alternately.

For example, when the carbon nanotube sources 310 are arranged in two rows, the irradiation of the carbon nanotube sources 310 in the first row is sequentially performed, and then the carbon nanotube sources 310 in the first row are in a standby state and the irradiation of the carbon nanotube sources 310 in the second rows waiting to be used is sequentially performed, thereby allowing radiation therapy.
treatment time is shortened and intensive radiation therapy is possible by performing radiation therapy alternately using the carbon nanotube sources 310 in the first and second rows in this way.

Meanwhile, the present invention may provide a controller 20 for selectively controlling the carbon nanotube sources 310 or checking a patient's condition using entered patient information to determine a treatment direction, and then performing radiation therapy by controlling the operation of the low-dose radiation therapy device 10.

In the present invention, a selective treatment mode may be set according to the type of disease and a patient's condition using the controller 20.

For example, there is a need to vary the intensity and irradiation band of radiation used for the treatment of each of skin cancer, benign keloids, osteoarthritis, heterotopic bone ossification, psoriasis, atopy, dementia, Parkinson's disease, animal cancer treatment and acute and chronic pain in skeletal muscle areas and intractable inflammatory diseases. For this, among the plural carbon nanotube sources 310 divided by band, a carbon nanotube source 310 in a specific band may be selectively operated, or two or more carbon nanotube sources 310 may be simultaneously operated to provide complex treatment.

Accordingly, a variety of treatment modes may be set and operated in advance considering the patient's age or health condition, and the selective operation of multiple carbon nanotube sources and the total amount and number of irradiated radiation according to a set treatment mode may be complexly controlled by manipulating the controller 20.

In particular, the controller 20 checks patient's conditions based on previously entered patient information when a patient lies down on the treatment bed 100 and waits for treatment to determine a treatment direction, and then operates the treatment bed 100 according to the judgment of the controller 20 such that the patient is positioned at an optimal treatment position, and then operates the radiation gantry 300 to perform radiation therapy.

The controller 20 may be installed on the outside of the radiation gantry 300 for the convenient manipulation of an operator as shown in FIGS. 9 to 11.

In addition, as the carbon nanotube sources 310 used in the present invention, a carbon nanotube X-ray source including a metal emitter and a carbon nanotube (CNT) growing in the emitter may be used.

Here, the carbon nanotube (CNT) emits electrons using a field emission method and may be formed in an irregular shape upward on the top of a pad. Plasma Enhanced Chemical Vapor Deposition (PECVD) or Thermal Chemical Vapor Deposition (Thermal CVD) supplying hydrocarbon gas may be used as a CNT growth process.

As described above, the present invention can provide a low-dose radiation treatment system including a low-dose radiation therapy device configured to form a radiation gantry so that therapeutic radiation is irradiated toward the patient's body by installing a plurality of carbon nanotube sources around the inner periphery of a ring-shaped gate, wherein an optimized radiation treatment environment tailored to a patient is provided by checking a patient's condition and setting a treatment direction using patient information entered through a controller, and then controlling the operation of the low-dose radiation therapy device to perform radiation therapy and adjust the size of radiation energy irradiated from the radiation therapy device according to the patient's individual characteristics and a treatment purpose, an angle at which the radiation is irradiated, and the radiation dose.

**In** addition, the present invention is advantageous for the treatment of chronic inflammation such as dementia sensitive to radiation exposure wherein a plurality of carbon nanotube sources are installed around the inner periphery of a radiation gantry, the carbon nanotube sources are distributed by band in a 100 to 300 kVp range, and a controller selectively uses radiation in a specific area depending upon a patient's disease type, can perform patient-tailored treatment by establishing a precise radiation treatment plan, is safe because it reduces the risk of radiation exposure, and can be used to treat benign keloids, arthritis, heterotopic bone ossification, psoriasis, atopy, dementia, Parkinson's disease, animal cancer, acute and chronic pain in skeletal muscle areas and intractable inflammatory diseases as well as skin cancer.

The present invention has been described with reference to an embodiment shown in the attached drawings, but this is merely illustrative, and those skilled in the art will recognize that various modifications and other equivalent embodiments are possible therefrom. Therefore, the true scope of protection of the present invention should be determined only by the appended claims.

## Claims

1. A low-dose radiation treatment system for dementia or chronic inflammation treatment based on a photon energy level, comprising:
a low-dose radiation therapy device wherein a treatment bed where a patient receives treatment while lying down is formed on a device body and a plurality of carbon nanotube sources are installed around the inner periphery of a ring-shaped gate of the device body to form a radiation gantry so that therapeutic radiation is irradiated toward the patient's body; and
a controller configured to check a patient's condition using entered patient information, to set a treatment direction and to perform radiation therapy by driving the treatment bed such that a patient is positioned at an optimal treatment position and by controlling the operation of the low-dose radiation therapy device,
wherein the plural carbon nanotube sources are installed around the inner periphery of the radiation gantry, and the carbon nanotube sources are distributed by band in a 100 to 300 kVp range, and the controller may selectively use radiation in a specific area depending upon a patient's disease type.

2. The low-dose radiation treatment system according to claim 1, wherein the controller controls a preset dose, energy intensity and a beam irradiation range and angle depending upon a patient's disease type.

3. The low-dose radiation treatment system according to claim 1, wherein a plurality of carbon nanotube sources are arranged in the radiation gantry the inner periphery, the carbon nanotube sources are distributed along the arc radius, and each of the carbon nanotube sources emits a radiation beam toward a concentric center of an arc.

4. The low-dose radiation treatment system according to claim 3, wherein the radiation gantry emits a dose of 10 to 100 cGy/min to the installed carbon nanotube sources, and the controller enables a preset dose to be irradiated depending upon a patient's disease type.

5. The low-dose radiation treatment system according to claim 3, wherein the plural carbon nanotube sources emit radiation beams sequentially or alternately.

6. The low-dose radiation treatment system according to one of claims 1 to 5, wherein radiation beam energy irradiated from the radiation gantry is set to a pulsed beam energy of 100~300 kVp and 3mA, and a radiation time (beam on-off time) of the radiation beam is set to 1 minute or less.

7. The low-dose radiation treatment system according to claim 1, wherein the low-dose radiation therapy device comprises:
a treatment bed where a patient lies down and receives treatment;
a device body where the treatment bed is installed to reciprocate in a longitudinal direction; and
a radiation gantry installed in an inner periphery direction of the gate such that therapeutic radiation is irradiated toward patient's body,
wherein a ring-shaped gate is formed on an upper side of the device body so that the bed passes through a center thereof.

8. The low-dose radiation treatment system according to claim 7, further comprising a rotator for rotating the radiation gantry around the patient's body to change a radiation irradiation position.

9. The low-dose radiation treatment system according to claim 7, wherein carbon nanotube sources are arranged in multiple rows around the inner periphery of the gate of the radiation gantry, wherein the carbon nanotube source arrays operate alternatively.
